(19) Europäisches Patentamt / European Patent Office / Office européen des brevets

(11) **EP 2 870 183 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**27.11.2024   Patentblatt 2024/48**

(45) Hinweis auf die Patenterteilung:
**01.12.2021   Patentblatt 2021/48**

(21) Anmeldenummer: **13730884.7**

(22) Anmeldetag: **21.06.2013**

(51) Internationale Patentklassifikation (IPC):
*C08F 6/00* (2006.01)      *A61L 15/22* (2006.01)
*A61L 15/60* (2006.01)      *C08J 3/24* (2006.01)
*A61L 15/24* (2006.01)      *A61L 15/42* (2006.01)

(52) Gemeinsame Patentklassifikation (CPC):
C08J 3/245; A61L 15/22; A61L 15/60; C08F 6/008;
C08J 2333/02

(86) Internationale Anmeldenummer:
**PCT/EP2013/063030**

(87) Internationale Veröffentlichungsnummer:
**WO 2014/005860 (09.01.2014 Gazette 2014/02)**

(54) **VERFAHREN ZUR HERSTELLUNG WASSERABSORBIERENDER POLYMERPARTIKEL MIT VERBESSERTEM EIGENSCHAFTSPROFIL**

METHOD FOR PRODUCING WATER-ABSORBENT POLYMER PARTICLES WITH IMPROVED PROPERTIES

PROCÉDÉ DE PRODUCTION DE PARTICULES POLYMÈRES ABSORBANT L'EAU, À PROPRIÉTÉS AMÉLIORÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:   03.07.2012   US 201261667476 P
03.07.2012   EP 12174828

(43) Veröffentlichungstag der Anmeldung:
**13.05.2015   Patentblatt 2015/20**

(73) Patentinhaber: **BASF SE**
**67056 Ludwigshafen am Rhein (DE)**

(72) Erfinder:
• **BAUDUIN, Christophe**
**68723 Plankstadt (DE)**
• **DANIEL, Thomas**
**67165 Waldsee (DE)**
• **KARIM, Asif**
**68159 Mannheim (DE)**

(74) Vertreter: **BASF IP Association**
**BASF SE**
**G-FLP-C006**
**67056 Ludwigshafen (DE)**

(56) Entgegenhaltungen:
WO-A1-2010/057912      WO-A1-2010/057912
WO-A1-2010/149735      WO-A1-2012/045705
WO-A1-2012/045705      WO-A1-2012/045705
WO-A1-2012/143215      US-A1- 2011 059 329
US-A1- 2011 059 329

EP 2 870 183 B2

# EP 2 870 183 B2

**Beschreibung**

Die vorliegende Erfindung betrifft ein Verfahren zur Herstellung wasserabsorbierender Polymerpartikel mit verbessertem Eigenschaftsprofil, umfassend thermische Oberflächennachvernetzung in Gegenwart eines Salzes aus einem polvalenten Metallkation und einem komplexierenden Anion und die anschließende Nachbehandlung, wobei die Nachbehandlung die Beschichtung mit einem Salz aus einem polvalenten Metallkation und einem nicht-komplexierenden Anion und die Nachbefeuchtung mit erneuter Trocknung umfasst.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden oft auch als "absorbent resin", "superabsorbents ", "superabsorbent polymer", "absorbent polymer", "absorbent gelling material", "hydrophilic polymer", "Hydrogele" oder "Superabsorber" bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Flüssigkeitsweiterleitung (SFC) Gelbettpermeabilität (GBP) und Absorption unter einem Druck von 49.2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7ps i) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der wasserabsorbierenden Polymerpartikel kovalente Bindungen bilden können.

WO 2012/045705 A1 offenbart ein Verfahren zur Herstellung thermisch oberflächennachvernetzter wasserabsorbierender Polymerpartikel, wobei die wasserabsorbierenden Polymerpartikel vor, während oder nach der thermischen Oberflächennachvernetzung mit mindestens einem mehrwertigen Metallsalz beschichtet werden und das mehrwertige Metallsalz das Anion der Glykolsäure oder das Anion eines Glykolsäurederivats enthält. Diese Schrift lehrt keine weitere Trocknung im Anschluss an die Zugabe einer Metallsalzlösung nach der thermischen Oberflächennachvernetzung oder im Anschluss an die Zugabe von Wasser im Kühler.

WO 2012/107432 A1 offenbart ein Verfahren zur Herstellung wasserabsorbierender Polymerpartikel mit hoher Anquellgeschwindigkeit, umfassend die Schritte Polymerisation, Trocknung, Mahlung, Klassierung und thermische Oberflächennachvernetzung, Nachbefeuchtung und erneute Trocknung.

WO 2010/149735 A1 lehrt die Zugabe von Aluminiumsalz zu Superabsorbern, wobei das Anion aus einer Liste zu wählen ist, die sowohl komplexierende wie nicht komplexierende Anionen enthält. Die Zugabe kann vor, während oder nach der Oberflächennachvernetzung erfolgen, vorzugsweise erfolgt sie davor. D2 lehrt weiterhin die Möglichkeit der Nachbefeuchtung nach der Oberflächennachvernetzung zur Einstellung des erwünschten Wassergehalts. Diese Schrift lehrt keine doppelte Zugabe von Aluminiumsalz, einmal mit komplexierendem und einmal mit nicht komplexierendem Anion, und keine Trocknung nach Nachbefeuchtung.

US 2011/059329 A1 betrifft ein Verfahren zur Herstellung von Superabsorbern, bei dem Tropfen einer Monomerlösung in einer umgebenden Gasphase polymerisiert werden, wodurch die Superabsorberpartikel eine mittlere Spherizität von 0,86 bis 0,99 erreichen, im Gegensatz zu Superabsorbern, die nach Lösungspolymerisation gemahlen und klassiert werden und dadurch eine mittlere Spherizität der Partikel von 0,72 bis 0,78 erreichen. Diese Schrift lehrt weiterhin, bei der Oberflächennachvernetzung Salze mehrwertiger Kationen zuzusetzen, wobei in der Liste möglicher Anionen nicht zwischen komplexierend und nicht komplexierend unterschieden wird. Sie lehrt weiterhin, mehrwertige Kationen nach der Oberflächennachvernetzung zuzusetzen, wobei dort als "schwach komplexierend" bezeichnete Anionen bevorzugt sind. Nach der Lehre von D3 wird Mahlung und Siebung nach der Polymerisation erfindungsgemäß vermieden und es erfolgt ferner keine Trocknung nach der letzten Zugabe von Wasser.

WO 2010/057912 A1 offenbart ebenfalls ein Verfahren zur Vertropfungspolymerisation. Dabei werden die entstehenden Polymerpartikel mit einem Permeabilitätsverbesserer beschichtet und/oder mit Wasserdampf vorbehandelt. Als Permeabilitätsverbesserer können mehrwertige Metallsalze, auch Gemische solcher Salze, eingesetzt werden, wobei als Anionen eine nicht nach komplexierend oder nicht komplexierend differenzierte Liste angegeben wird. Die Vorbehandlung mit Wasserdampf erfolgt gegebenenfalls vor der Zugabe des Permeabilitätsverbesserers Diese Schrift schweigt zu Mahlung, Siebung, Oberflächennachvernetzung, zweimaliger Zugabe eines mehrwertigen Metallsalzes, Nachbefeuchtung oder Trocknung nach Nachbefeuchtung.

Aufgabe der vorliegenden Erfindung war die Bereitstellung eines Verfahrens zur Herstellung wasserabsorbierender Polymerpartikel mit verbessertem Eigenschaftsprofil.

Gelöst wurde die Aufgabe durch ein Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
wobei das erhaltene Polymergel getrocknet, gemahlen und klassiert wird, die klassierten Polymerpartikel mit
f) mindestens einem kovalenten Oberflächennachvernetzer und
g) mindestens einem Salz aus einem polyvalenten Metallkation und einem komplexierenden Säureanion

thermisch oberflächennachvernetzt werden, dadurch gekennzeichnet, dass die oberflächennachvernetzten Polymerpartikel anschließend Nachbehandelt werden und die Nachbehandlung die Schritte

i) Beschichtung mit mindestens einem Salz aus einem polyvalenten Metallkation und einem nicht-komplexierenden Säuranion,
ii) Erhöhung des Feuchtgehalts um 1 bis 150 Gew.-% und
iii) Trocknung nach der Erhöhung des Feuchtgehalts

umfasst.

Die Verfahrensschritte i) und ii) können in beliebiger Reihenfolge durchgeführt werden. Vorzugsweise wird allerdings Schritt i) vor Schritt ii) durchgeführt.

Geeignete komplexierende Säureanionen für das mindestens eine Salz g) sind Carbonsäureanionen, die neben der Carbonsäure-Gruppe mindestens eine zur Komplexierung geeignete funktionelle Gruppe aufweisen. Derartige funktionelle Gruppen weisen freie Elektronenpaare auf ohne selber zum Ladungsausgleich des polyvalenten Metallkations beizutragen, wie beispielsweise Hydroxy- und Amino-Gruppen. Bevorzugte Säureanionen für das mindestens eine Salz g) sind Glykolat, Glycinat, Laktat, Alanat, Citrat, Tartrat, Tartronat und Glycerat.

Geeignete polyvalente Metallkationen für das Salz g) sind beispielsweise zweiwertige Kationen, wie $Zn^{2+}$, $Mg^{2+}$, $Ca^{2+}$, $Fe^{2+}$ und $Sr^{2+}$, dreiwertige Kationen, wie $Al^{3+}$, $Fe^{3+}$, $Cr^{3+}$, und $Mn^{3+}$, vierwertige Kationen, wie $Ti^{4+}$ und $Zr^{4+}$. Bevorzugte polyvalente Metallkationen sind $Al^{3+}$, $Ti^{4+}$ und $Zr^{4+}$. Ganz besonders bevorzugtes Salz g) ist Aluminiumlaktat.

Die Einsatzmenge an polyvalentem Metallkation im Salz g) beträgt vorzugsweise 0,001 bis 1,5 Gew.-%, besonders bevorzugt 0,005 bis 1 Gew.-%, ganz besonders bevorzugt 0,02 bis 0,8 Gew.-%. jeweils bezogen auf die Polymerpartikel.

Geeignete nicht-komplexierende Säureanionen für das Salz in Schritt i) sind organische Säureanionen, die neben der Säure-Gruppe keine zur Komplexierung geeignete funktionelle Gruppe aufweisen, oder anorganische Säureanionen. Besonders bevorzugte Säureanionen für das mindestens eine Salz in Schritt i) sind Formiat, Acetat, Propionat, Methylsulfonat, Sulfat und Chlorid.

Geeignete polyvalente Metallkationen für das Salz in Schritt i) sind beispielsweise zweiwertige Kationen, wie $Zn^{2+}$, $Mg^{2+}$, $Ca^{2+}$, $Fe^{2+}$ und $Sr^{2+}$, dreiwertige Kationen, wie $Al^{3+}$, $Fe^{3+}$, $Cr^{3+}$, und $Mn3+$, vierwertige Kationen, wie $Ti^{4+}$ und $Zr^{4+}$. Bevorzugte polyvalente Metallkationen sind $Al^{3+}$, $Ti^{4+}$ und $Zr^{4+}$. Ganz besonders bevorzugte Salze in Schritt i) sind Aluminiumsulfat, Natriumalaun und Kaliumalaun.

Die Einsatzmenge an polyvalentem Metallkation im Salz in Schritt i) beträgt vorzugsweise 0,001 bis 1,5 Gew.-%, besonders bevorzugt 0,005 bis 1 Gew.-%, ganz besonders bevorzugt 0,02 bis 0,8 Gew.-%. jeweils bezogen auf die Polymerpartikel.

Der Feuchtegehalt in Schritt ii) wird um vorzugsweise 2,5 bis 100 Gew.-%, besonders bevorzugt 5 bis 50 Gew.-%, ganz besonders 10 bis 25 Gew.-%, erhöht (Nachbefeuchtung). Die Art und Weise, mit der der Feuchtegehalt erhöht wird, unterliegt keiner Beschränkung. Beispielsweise können die wasserabsorbierenden Polymerpartikel mit Wasser in flüssiger oder gasförmiger Form in Kontakt gebracht werden, beispielsweise durch Aufsprühen oder durch Durchlüftung mit feuchten Gasen (Luft, Stickstoff, usw.). Alternativ können zerstoßenes Eis oder bereits feuchte wasserabsorbierende Polymerpartikel untergemischt werden. Es sind auch Kombinationen unterschiedlicher Zugabeformen möglich, beispielsweise eine wässrige Lösung des Salzes aus polyvalenten Metallkation und nicht-komplexierenden Säureanion und Wasserdampf.

Die Produkttemperatur beträgt während der Erhöhung des Wassergehalts beispielsweise 0 bis 140°C, vorzugsweise 20 bis 120°C, besonders bevorzugt 50 bis 100°C, ganz besonders bevorzugt 60 bis 90°C.

Die Verweilzeit zwischen Erhöhung des Wassergehalts und anschließender Trocknung ist unkritisch und beträgt beispielsweise weniger als 10 Tage, vorzugsweise weniger als 5 Tage, bevorzugt weniger als einen Tag, besonders bevorzugt weniger als 6 Stunden, ganz besonders bevorzugt weniger als 2 Stunden.

Anschließend werden die wasserabsorbierenden Polymerpartikel bei Temperaturen von vorzugsweise weniger als 150°C, besonders bevorzugt weniger als 130°C, ganz besonders bevorzugt weniger als 110°, bis zu einem Feuchtegehalt von vorzugsweise weniger als 10 Gew.-%, besonders bevorzugt weniger als 7 Gew.-%, ganz besonders bevorzugt weniger als 5 Gew.-%, getrocknet.

Die anschließende Trocknung kann statisch oder dynamisch durchgeführt werden, d.h. die wasserabsorbierenden Polymerpartikel werden dabei bewegt, beispielsweise gerührt, oder nicht. Vorzugsweise wird dynamisch getrocknet. Der Druck bei der Trocknung ist ebenfalls unkritisch und entspricht beispielsweise dem Umgebungsdruck oder weniger (Unterdruck). Es ist aber auch möglich die wasserabsorbierenden Polymerpartikel zur Trocknung mit einem trockenen Gas (Luft, Stickstoff, usw.) zu durchlüften.

In einer bevorzugten Ausführungsform der vorliegenden Erfindung wird bei der erfindungsgemäßen Trocknung der gewünschte Feuchtegehalt für das Endprodukt eingestellt.

Der vorliegenden Erfindung liegt die Erkenntnis zugrunde, dass der Vortex und die Permeabilität, d.h. die Flüssigkeitsweiterleitung (SFC) und die Gelbettpermeabilität (GBP), wasserabsorbierender Polymerpartikel gleichzeitig verbessert werden kann, indem die thermische Oberflächennachvernetzung in Gegenwart eines Salzes aus einem polyvalenten Metallkation und einem komplexierenden Säureanion durchgeführt wird und die oberflächennachvernetzten Polymerpartikel anschließend mit einem Salz aus einem polyvalenten Metallkation und einem nicht-komplexierenden Säureanion beschichtet, gequollen und wieder getrocknet werden. Die Beschichtung der oberflächennachvernetzten Polymerpartikel mit dem Salz aus einem polyvalenten Metallkation und einem nicht-komplexierenden Säureanion kann auch nach Quellung und Trocknung durchgeführt werden, d.h. die Reihenfolge der beiden Nachbehandlungsschritte ist nicht wesentlich.

Im Folgenden wird die Herstellung der wasserabsorbierenden Polymerpartikel näher erläutert:
Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure. Gut geeignet sind auch Monomere a), die aus nachwachsenden Rohstoffen hergestellt werden.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Dioder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 20-fach ethoxylierten Glyzerins. Ganz besonders bevorzugt sind Dioder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins .

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a).

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise Dinatrium-2-hydroxy-2-sulfonatoazetat oder ein Gemisch aus Dinatrium-2-hydroxy-2-sulfinatoazetat, Dinatrium-2-hydroxy-2-sulfonatoazetat und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomerlösung oder -suspension wird polymerisiert. Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das erhaltene Polymergel wird getrocknet. Die Trockner unterliegen keiner Beschränkung. Die Trocknung des Polymergels wird aber vorzugsweise mit einem Bandtrockner durchgeführt bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird gemahlen und klassiert, wobei zur Mahlung üblicherweise einoder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Flüssigkeitsweiterleitung (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel werden zur Verbesserung der Eigenschaften thermisch oberflächennachvernetzt. Geeignete Oberflächennachvernetzer f) sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer f) beschrieben.

Bevorzugte Oberflächennachvernetzer f) sind Ethylenkarbonat, Propylenkarbonat, Glyzerinkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer f) sind 2-Hydroxyethyloxazolidin-2-on, Oxazolidin-2-on und 1,3-Propandiol.

Weiterhin können auch Oberflächennachvernetzer f) eingesetzt werden, die zusätzliche polymerisierbare ethylenisch ungesättigte Gruppen enthalten, wie in DE 37 13 601 A1 beschrieben

Die Menge an Oberflächennachvernetzer f) beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

Die thermische Oberflächennachvernetzung wird in Gegenwart mindestens eines Salzes g) aus einem polyvalenten Metallkation und einem komplexierenden Säureanion durchgeführt. Das Salz g) aus einem polyvalenten Metallkation und einem komplexierenden Säureanion kann vor oder während der thermischn Oberflächennachvernetzung auf die Partikeloberfläche aufgebracht werden.

Die Oberflächennachvernetzung wird üblicherweise so durchgeführt, dass eine Lösung des Oberflächennachvernetzers f) auf die getrockneten Polymerpartikel aufgesprüht wird. Im Anschluss an das Aufsprühen werden die mit Oberflächennachvernetzer f) beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers f) wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer f) werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers f) in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland) und Nara Paddle Dryer (NARA Machinery Europe; Frechen; Deutschland). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Oberflächennachvernetzungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Nach der thermischen Oberflächennachvernetzung wird die erfindungsgemäße Nachbehandlung durchgeführt.

Anschließend können die oberflächennachvernetzten und nachbehandelten Polymerpartikel erneut klassiert werden, wobei zu kleine und/oder zu große Polymerpartikel abgetrennt und in das Verfahren rückgeführt werden.

Die Polymerpartikel können zur weiteren Verbesserung der Eigenschaften zusätzlich beschichtet und erneut nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Flüssigkeitsweiterleitung (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind die gemäß dem erfindungsgemäßen Verfahren erhältlichen wasserabsorbierenden Polymerpartikel.

Ein weiterer Gegenstand der vorliegenden Erfindung sind wasserabsorbierende Polymerpartikel, erhältlich durch Polymerisation einer Monomerlösung oder -suspension zu einem Polymergel, Trocknung, Mahlung und Klassierung des Polymergels zu Polymerpartikeln und thermischer Oberflächennachvernetzung der Polymerpartikel, wobei die wasserabsorbierenden Polymerpartikel einen Feuchtegehalt von weniger als 10 Gew.-%, eine Zentrifugenretentionskapazität von mindestens 15 g/g, eine Flüssigkeitsweiterleitung von mindestens $80 \times 10^{-7}$ cm³s/g, eine Gelbettpermeabilität von mindestens 30 Darcies und einen Vortex von weniger als 70s aufweisen.

Die erfindungsgemäßen wasserabsorbierenden Polymerpartikel weisen typischerweise eine hohe Flüssigkeitsweiterleitung (SFC) von mindestens $100 \times 10^{-7}$ cm³s/g, ein hohe Gelbettpermeabilität (GBP) und einen niedrigen Vortex auf, beispielsweise eine Flüssigkeitsweiterleitung (SFC) von vorzugsweise mindestens $130 \times 10^{-7}$ cm³s/g, besonders bevorzugt von $150$ bis $250 \times 10^{-7}$ cm³s/g, eine Gelbettpermeabilität (GBP) von vorzugsweise mindestens 40 Darcies, besonders bevorzugt von mindestens 45 Darcies, ganz besonders bevorzugt von 50 bis 100 Darcies, und einen Vortex von vorzugsweise weniger als 65 s, besonders bevorzugt von weniger als 62s, ganz besonders bevorzugt von 40 bis 60s.

Die erfindungsgemäßen wasserabsorbierenden Polymerpartikel weisen eine Zentrifugenretentionskapazität (CRC) von typischerweise mindestens 15 g/g, vorzugsweise mindestens 18 g/g, bevorzugt mindestens 20 g/g, besonders bevorzugt mindestens 22 g/g, ganz besonders bevorzugt 23 bis 40 g/g, auf. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die erfindungsgemäßen wasserabsorbierenden Polymerpartikel weisen eine Absorption unter einem Druck von 49,2 g/cm² von typischerweise mindestens 15 g/g, vorzugsweise mindestens 18 g/g, bevorzugt mindestens 20 g/g, besonders bevorzugt mindestens 22 g/g, ganz besonders bevorzugt 23 bis 40 g/g, auf. Die Absorption unter einem Druck von 49,2 g/cm² wird analog der von der EDANA empfohlenen Testmethode Nr. WSP 242.2-05 "Absorption Under Pressure, Gravimetric Determination" bestimmt, wobei statt eines Drucks von 21,0 g/cm² ein Druck von 49,2 g/cm² eingestellt wird.

Die erfindungsgemäßen wasserabsorbierenden Polymerpartikel weisen einen Feuchtegehalt von vorzugsweise weniger als 10 Gew.-%, besonders bevorzugt weniger als 8 Gew.-%, ganz besonders bevorzugt von 0,5 bis 6 Gew.-%, auf, wobei der Feuchtegehalt gemäß der von der EDANA empfohlenen Testmethode Nr. WSP 230.2-05 "Mass Loss Upon Heating" bestimmt wird.

Ein weiterer Gegenstand der vorliegenden Erfindung sind Hygieneartikel, enthaltend erfindungsgemäße wasserabsorbierende Polymerpartikel.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Die wasserabsorbierenden Polymerpartikel werden mittels der nachfolgend beschriebenen Testmethoden geprüft.

Die mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugene Plasky 157, 1030 Brussels, Belgium, www.edana.org) und INDA (1100 Crescent Green, Cary, NC 27518, U.S.A., www.inda.org). Diese Veröffentlichung ist sowohl von EDANA als auch von INDA erhältlich.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Feuchtegehalt

Der Feuchtegehalt der wasserabsorbierenden Polymerpartikel wird gemäß der von der EDANA empfohlenen Testmethode Nr. WSP 230.2-05 "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 0,0 g/cm$^2$ (Absorption under Load)

Die Absorption unter einem Druck von 0,0 g/cm$^2$ (AUL0.0psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 0,0 g/cm$^2$ (AUL0.0psi) eingestellt wird.

Absorption unter einem Druck von 21,0 g/cm$^2$ (Absorption under Load)

Die Absorption unter einem Druck von 21,0 g/cm$^2$ (AUL0.3psi) wird gemäß der von der EDANA empfohlenen Testmethode Nr. WSP 242.2-05 "Absorption Under Pressure, Gravimetric Determination" bestimmt.

Absorption unter einem Druck von 49,2 g/cm$^2$ (Absorption under Load)

Die Absorption unter einem Druck von 49,2 g/cm$^2$ (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 49,2 g/cm$^2$ (AUL0.7psi) eingestellt wird.

Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

Anquellgeschwindigkeit (Free Swell Rate)

Zur Bestimmung der Anquellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$\text{FSR [g/g s] = W2/(W1xt)}$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Vortex

In ein 100 ml-Becherglas, welches ein Magnetrührstäbchen der Größe 30 mm x 6 mm enthält, werden 50,0 ml ± 1,0 ml einer 0,9 Gew.-%igen wässrigen Kochsalzlösung gegeben. Mit Hilfe eines Magnetrührers wird die Kochsalzlösung bei 600 Upm gerührt. Es werden dann möglichst schnell 2,000 g ± 0,010 g wasserabsorbierende Polymerpartikel zugegeben, und die Zeit gemessen, die vergeht, bis die Rührtraube durch die Absorption der Kochsalzlösung durch die wasserabsorbierenden Polymerpartikel verschwindet. Dabei kann der ganze Inhalt des Becherglases sich als einheitliche Gelmasse immer noch drehen, aber die Oberfläche der gelierten Kochsalzlösung darf keine individuellen Turbulenzen mehr zeigen. Die benötigte Zeit wird als Vortex berichtet.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC [cm}^3\text{s/g] = (Fg(t=0)xL0)/(dxAxWP),}$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

Gelbettpermeabilität (Gel Bed Permeability)

Die Gelbettpermeabilität (GBP) einer gequollenen Gelschicht unter Druckbelastung von 0,3 psi (2070 Pa) wird, wie in US 2005/0256757 beschrieben (Absätze [0061] und [0075]), als Gel-Bed-Permeability einer gequollenen Gelschicht aus wasserabsorbierenden Polymerpartikeln bestimmt.

Beispiele

Herstellung des Grundpolymers

Beispiel 1

Ein Grundpolymer wurde analog dem in der WO 01/38402 A1 beschriebenen kontinuierlichen Kneterverfahren in einem Reaktor vom Typ List Contikneter mit einem Volumen 6,3m$^3$ (LIST AG, Arisdorf, CH) hergestellt. Dazu wurde Acrylsäure mit Natronlauge kontinuierlich neutralisiert und mit Wasser verdünnt, so dass der Neutralisationsgrad der Acrylsäure 69 mol-% und der Feststoffgehalt (= Natriumacrylat und Acrylsäure) dieser Lösung ca. 40,0 Gew.-% betrug. Als Vernetzer wurde mit Acrylsäure verestertes und insgesamt 3-fach ethoxyliertes Glycerintriacrylat eingesetzt (Gly-3 EO-TA), dass gemäß US 2005/0176910 hergestellt wurde, und zwar in einer Menge von 0,348 Gew.-% bezogen auf Acrylsäuremonomer. Der Vernetzer wurde dem Monomerstrom kontinuierlich zugemischt. Für die Berechnung des Acrylsäuremonomergehaltes wurde dabei das enthaltene Natriumacrylat rechnerisch als Acrylsäure berücksichtigt. Die Initiation erfolgte durch ebenfalls kontinuierliche Zumischung wässriger Lösungen der Initiatoren Natriumpersulfat (0,195 Gew.-% bezogen auf Acrylsäuremonomer), Wasserstoffperoxid (0,002 Gew.-% bezogen auf Acrylsäuremonomer) und Ascorbinsäure (0,0031 Gew.-% bezogen auf Acrylsäuremonomer).

Das erhaltene Polymergel wurde auf einem Bandtrockner getrocknet, anschließend der Trocknerkuchen gebrochen, mittels eines Walzenstuhls gemahlen und schließlich auf eine Korngröße von 150 bis 850 μm abgesiebt.

Das so hergestellte Grundpolymer wies folgende Eigenschaften auf:

CRC = 36,0 g/g
Extrahierbare (16 h) = 14,0 Gew.-%

Partikelgrößenverteilung

| >850 μm | < 0,1 Gew.-% |
|---|---|
| 600-850 μm | 3,61 Gew.-% |
| 300-600 μm | 77,55 Gew.-% |
| 150-300 μm | 18,8 Gew.-% |
| <150 μm | < 0,1 Gew.-% |

Beispiel 2

Ein weiteres Grundpolymer analog dem in der WO 01/38402 A1 beschriebenen kontinuierlichen Kneterverfahren in einem Reaktor vom Typ List Contikneter mit einem Volumen 6,3m$^3$ (LIST AG, Arisdorf, CH) hergestellt. Dazu wurde Acrylsäure mit Natronlauge kontinuierlich neutralisiert und mit Wasser verdünnt, so dass der Neutralisationsgrad der Acrylsäure 72 mol-% und der Feststoffgehalt (= Natriumacrylat und Acrylsäure) dieser Lösung ca. 38,8 Gew.-% betrug. Als Vernetzer wurde Gly-3EO-TA in einer Menge von 0,484 Gew.-% bezogen auf Acrylsäuremonomer eingesetzt. Der Vernetzer wurde dem Monomerstrom kontinuierlich zugemischt. Die Initiation erfolgte durch ebenfalls kontinuierliche Zumischung wässriger Lösungen der Initiatoren Natriumpersulfat (0,14 Gew.-% bezogen auf Acrylsäuremonomer), Wasserstoffperoxid (0,001 Gew.-% bezogen auf Acrylsäuremonomer) und Ascorbinsäure (0,002 Gew.-% bezogen auf Acrylsäuremonomer).

Das erhaltene Polymergel wurde auf einem Bandtrockner getrocknet, anschließend der Trocknerkuchen gebrochen, auf einem Walzenstuhl gemahlen und schließlich auf eine Korngröße von 150 bis 850 μm abgesiebt.

Das so hergestellte Grundpolymer wies folgende Eigenschaften auf:

CRC = 33,6 g/g
Extrahierbare (16 h) = 12,2 Gew.-%

Partikelgrößenverteilung

| >850 μm | 0,02 Gew.-% |
|---|---|
| 600-850 μm | 26,1 Gew.-% |
| 300-600 μm | 48,3 Gew.-% |
| 150-300 μm | 24,9 Gew.-% |
| <150 μm | <0,1 Gew.-% |

Oberflächennachvernetzung des Grundpolymers

Beispiel 3

In einem Schugi®-Flexomix Typ 100 D (Hosokawa-Micron B.V., Doetichem, Niederlande) mit gravimetrischer Eindosierung und kontinuierlicher Massenfluss-kontrollierter Flüssigkeitsdosierung über eine Flüssigkeitsdüse wurde Grundpolymer aus Beispiel 1 mit einer Oberflächennachvernetzungslösung besprüht. Die Oberflächennachvernetzerlösung war ein Gemisch aus 0,07 Gew.-% N-(2-Hydroxyethyl)-oxazolidinon, 0,07 Gew.-% 1,3-Propandiol, 0,50 Gew.-% Aluminiumtrilaktat, 0,70 Gew.-% Propylenglykol, 1,00 Gew.-% Isopropanol und 2,22 Gew.-% Wasser, jeweils bezogen auf das Grundpolymer.

Das feuchte Grundpolymer wurde direkt aus dem Schugi®-Flexomix fallend in einen NARA-Paddle-Dryer® Typ NPD 1.6 W (GMF Gouda, Waddinxveen, Niederlande) überführt. Die Durchsatzrate an Grundpolymer betrug 60 kg/h (trocken) und die Produkttemperatur des mit Dampf beheizten Trockners am Trocknerausgang betrug ca. 188°C. Dem Trockner war ein Kühler nachgeschaltet, der das Produkt rasch auf ca. 50°C abkühlte. Die Verweilzeit im Trockner wurde über die konstante Durchsatzrate des Grundpolymers sowie die Wehrhöhe von 70% vorgegeben und betrug ca. 60 Minuten. Die notwendige Verweilzeit wird durch Vorversuche bestimmt mit deren Hilfe die konstante Dosierrate ermittelt wird, die zum gewünschten Eigenschaftsprofil führt. Dies ist im kontinuierlichen Prozess notwendig, da die sich die Schüttdichte während der Reaktionstrocknung stetig verändert. Die Eigenschaften des erhaltenen Polymers sind in der Tabelle 1 enthalten.

Beispiel 4

In einem Schugi®-Flexomix Typ 100 D (Hosokawa-Micron B.V., Doetichem, Niederlande) mit gravimetrischer Eindosierung und kontinuierlicher Massenfluss-kontrollierter Flüssigkeitsdosierung über eine Flüssigkeitsdüse wurde Grundpolymer aus Beispiel 2 mit einer Oberflächennachvernetzungslösung besprüht. Die Oberflächennachvernetzerlösung war ein Gemisch aus 0,11 Gew.-% Denacol® EX810 (Ethylenglykoldiglycidylether), 0,26 Gew.-% Aluminiumsulfat, 1,00 Gew.-% Propylenglykol und 2,00 Gew.-% Wasser, jeweils bezogen auf das Grundpolymer.
Das feuchte Grundpolymer wurde direkt aus dem Schugi®-Flexomix fallend in einen NARA-Paddle-Dryer® Typ NPD 1.6 W (GMF Gouda, Waddinxveen, Niederlande) überführt. Die Durchsatzrate an Grundpolymer betrug 60 kg/h (trocken) und die Produkttemperatur des mit Dampf beheizten Trockners am Trocknerausgang betrug ca. 180°C. Dem Trockner war ein Kühler nachgeschaltet, der das Produkt rasch auf ca. 50°C abkühlte. Die Verweilzeit im Trockner wurde über die konstante Durchsatzrate des Grundpolymers sowie die Wehrhöhe von 70% vorgegeben und betrug ca. 60 Minuten. Die notwendige Verweilzeit wird durch Vorversuche bestimmt mit deren Hilfe die konstante Dosierrate ermittelt wird, die zum gewünschten Eigenschaftsprofil führt. Dies ist im kontinuierlichen Prozess notwendig, da die sich die Schüttdichte während der Reaktionstrocknung stetig verändert. Die Eigenschaften des erhaltenen Polymers sind in der Tabelle 1 enthalten.

Tab. 1: Oberflächennachvernetzung des Grundpolymers

| Bsp. | CRC [g/g] | AUL0.7psi [g/g] | AUL0.3psi [g/g] | AUL0.0psi [g/g] | SFC [$10^{-7}cm^3g/s$] | GBP [darcies] | Vortex [s] | FSR [g/gs] |
|---|---|---|---|---|---|---|---|---|
| 3*) | 24,2 | 22,0 | 26,0 | 35,4 | 119 | 17 | 86 | 0,17 |
| 4*) | 29,7 | 21,8 | 28,3 | 42,8 | 45 | 22 | 95 | 0,20 |
| *) Vergleichsbeispiel | | | | | | | | |

Nachbehandlung nach der Oberflächennachvernetzung

Beispiel 5

In einem Pflugschar®-Schaufeltrockner Typ M5RMK mit 5 l Volumen (Gebr. Lödige Maschinenbau GmbH; Paderborn, Deutschland) wurden 1,2 kg trockenes Polymer aus Beispiel 3 vorgelegt. Anschließend wurde unter Rühren (60 U/min) innerhalb von ca. 120 Sekunden mittels einer mit Stickstoff betriebenen Zweistoffdüse eine Lösung aus 2 Gew.-% Wasser und 0,50 Gew.-% Aluminiumsulfat, jeweils bezogen auf das eingesetzte Polymer, aufgesprüht und insgesamt 15 Minuten gemischt. Abschließend wurde durch ein 850 μm Sieb abgesiebt, um Klumpen zu entfernen. Die Eigenschaften des erhaltenen Polymers sind in der Tabelle 2 enthalten.

Beispiel 6

In einem Pflugschar®-Schaufeltrockner Typ M5RMK mit 5 l Volumen (Gebr. Lödige Maschinenbau GmbH; Paderborn, Deutschland) wurden 1,2 kg trockenes Polymer aus Beispiel 4 vorgelegt. Anschließend wurde unter Rühren (60 U/min) innerhalb von ca. 120 Sekunden mittels einer mit Stickstoff betriebenen Zweistoffdüse eine Lösung aus 2 Gew.-% Wasser und 0,50 Gew.-% Aluminiumsulfat jeweils bezogen auf das eingesetzte Polymer, aufgesprüht und insgesamt 15 Minuten gemischt. Abschließend wurde durch ein 850 μm Sieb abgesiebt, um Klumpen zu entfernen. Die Eigenschaften des erhaltenen Polymers sind in der Tabelle 2 enthalten.

Beispiel 7

In einem Pflugschar®-Schaufeltrockner Typ M5RMK mit 5 l Volumen (Gebr. Lödige Maschinenbau GmbH; Paderborn, Deutschland) wurden 1,2 kg trockenes Polymer aus Beispiel 3 vorgelegt. Anschließend wurde unter Rühren (60 U/min) innerhalb von ca. 120 Sekunden mittels einer mit Stickstoff betriebenen Zweistoffdüse eine Lösung aus 2 Gew.-% Wasser und 0,50 Gew.-% Aluminiumtrilaktat jeweils bezogen auf das eingesetzte Polymer, aufgesprüht und insgesamt 15 Minuten gemischt. Abschließend wurde durch ein 850 μm Sieb abgesiebt, um Klumpen zu entfernen. Die Eigenschaften des erhaltenen Polymers sind in der Tabelle 2 enthalten.

Beispiel 8

Jeweils 100 g der oberflächennachvernetzten Polymerpartikel aus Beispiel 3 wurden 90 Minuten bei 90°C und einer relativen Luftfeuchte von 75% in einem Klimaschrank gelagert. Die Wasseraufnahme betrug während der Lagerung ca. 6 bis 8 Gew.-%. Anschließend wurde die Probe in eine 500ml Kunststoffflasche eingefüllt und 10 Minuten mittels eines Turbulamischers homogenisiert. Die Probe wurde in einen Rundkolben mit Strömungsbrechern eingefüllt und 15 Minuten bei 80°C unter Vakuum (27 bis 30 mbar) im Rotationverdampfer getrocknet. Anschließend wurde auf eine Partikelgröße von weniger als 850 μm abgesiebt. Die getrockneten Polymerpartikel wurden analysiert. Die Ergebnisse sind in der Tabelle 2 zusammengefasst.

Beispiel 9

Es wurde verfahren wie unter Beispiel 8. Statt Polymer aus Beispiel 3 wurde Polymer aus Beispiel 5 eingesetzt. Die Ergebnisse sind in der Tabelle 2 zusammengefasst.

Beispiel 10

Es wurde verfahren wie unter Beispiel 8. Statt Polymer aus Beispiel 3 wurde Polymer aus Beispiel 6 eingesetzt. Die Ergebnisse sind in der Tabelle 2 zusammengefasst.

Beispiel 11

Es wurde verfahren wie unter Beispiel 8. Statt Polymer aus Beispiel 3 wurde Polymer aus Beispiel 7 eingesetzt. Die Ergebnisse sind in der Tabelle 2 zusammengefasst.

Beispiel 12

In einem Pflugschar®-Schaufeltrockner Typ M5RMK mit 5 l Volumen (Gebr. Lödige Maschinenbau GmbH; Paderborn, Deutschland) wurden 1,2 kg trockenes Polymer aus Beispiel 8 vorgelegt. Anschließend wurde unter Rühren (60 U/min) innerhalb von ca. 120 Sekunden mittels einer mit Stickstoff betriebenen Zweistoffdüse eine Lösung aus 2 Gew.-% Wasser und 0,50 Gew.-% Aluminiumsulfat jeweils bezogen auf das eingesetzte Polymer, aufgesprüht und insgesamt 15 Minuten gemischt. Abschließend wurde durch ein 850 μm Sieb abgesiebt, um Klumpen zu entfernen. Die Eigenschaften des erhaltenen Polymers sind in der Tabelle 2 enthalten.

Tab.2: Nachbehandlung nach der Oberflächennachvernetzung

| Bsp. | CRC [g/g] | AUL0.7psi [g/g] | AUL0.3psi [g/g] | AUL0.0psi [g/g] | SFC [$10^{-7}cm^3g/s$] | GBP [darcies] | Vortex [s] | FSR [g/gs] | Feuchtegehalt [Gew.-%] |
|---|---|---|---|---|---|---|---|---|---|
| 5*) | 24,1 | 21,2 | 26,0 | 39,7 | 145 | 54 | 84 | 0,18 | 3,0 |
| 6*) | 28,4 | 20,4 | 26,8 | 45,1 | 54 | 88 | 80 | 0,22 | 4,0 |
| 7*) | 25,7 | 22,5 | 26,5 | 37,0 | 166 | 19 | 106 | 0,16 | 3,1 |
| 8*) | 25,3 | 22,9 | 26,6 | 36,1 | 130 | 16 | 90 | 0,19 | 5,2 |
| 9 | 24,9 | 21,5 | 26,4 | 39,6 | 187 | 53 | 58 | 0,22 | 1,6 |
| 10*) | 28,3 | 19,6 | 26,0 | 43,0 | 46 | 97 | 57 | 0,28 | 7,3 |
| 11*) | 25,3 | 21,0 | 25,6 | 35,9 | 182 | 21 | 74 | 0,19 | 6,3 |

(fortgesetzt)

| Bsp. | CRC [g/g] | AUL0.7psi [g/g] | AUL0.3psi [g/g] | AUL0.0psi [g/g] | SFC [$10^{-7}cm^3g/s$] | GBP [darcies] | Vortex [s] | FSR [g/gs] | Feuchtegehalt [Gew.-%] |
|---|---|---|---|---|---|---|---|---|---|
| 12 | 25,3 | 21,6 | 26,1 | 39,6 | 186 | 81 | 60 | 0,19 | 6,2 |

*) Vergleichsbeispiel

Tab. 3: Übersicht der Verfahrensbedingungen

| Beispiele | Schritt A | Schritt B | Schritt C |
|---|---|---|---|
| 1*) | NEIN | NEIN | NEIN |
| 2*) | NEIN | NEIN | NEIN |
| 3*) | JA: Al-Laktat | NEIN | NEIN |
| 4*) | NEIN: Al-Sulfat | NEIN | NEIN |
| 5*) | JA: Al-Laktat | JA: Al-Sulfat | NEIN |
| 6*) | NEIN: Al-Sulfat | JA: Al-Sulfat | NEIN |
| 7*) | JA: Al-Laktat | NEIN: Al-Laktat | NEIN |
| 8*) | JA: Al-Laktat | NEIN | JA |
| 9 | JA: Al-Laktat | JA: Al-Sulfat | JA |
| 10*) | NEIN: Al-Sulfat | JA: Al-Sulfat | JA |
| 11*) | JA: Al-Laktat | NEIN: Al-Laktat | JA |
| 12 | JA: Al-Laktat | JA: Al-Sulfat | JA |

Schritt A: Oberflächennachvernetzung in Gegenwart eines komplexierenden Säureanions
Schritt B: Nachbehandlung mit einem nicht-komplexierenden Säureanion
Schritt C: Erhöhung des Feuchtegehalts mit anschließender Trocknung
Al-Sulfat: Aluminiumsulfat
Al-Laktat: Aluminiumtrilaktat
*) Vergleichsbeispiel

Der Ergebnisse zeigen, dass nur bei Erfüllung aller erfindungswesentlichen Verfahrensschritte, d.h, Oberflächennachvernetzung in Gegenwart eines komplexierenden Säureanions, Nachbehandlung mit einem nicht-komplexierenden Säureanion und Erhöhung des Feuchtegehalts mit anschließender Trocknung, wasserabsorbierende Polymerpartikel mit hoher Flüssigkeitsweiterleitung (SFC), hoher Gelbettpermeabilität (GBP) und niedrigem Vortex erhalten werden.

**Patentansprüche**

1.  Verfahren zur Herstellung wasserabsorbierender Polymerpartikel durch Polymerisation einer Monomerlösung oder -suspension, enthaltend

    a) mindestens ein ethylenisch ungesättigtes, säuregruppentragendes Monomer, das zumindest teilweise neutralisiert sein kann,
    b) mindestens einen Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,
    wobei das erhaltene Polymergel getrocknet, gemahlen und klassiert wird, die klassierten Polymerpartikel mit

    f) mindestens einem kovalenten Oberflächennachvernetzer und

g) mindestens einem Salz aus einem polyvalenten Metallkation und einem komplexierenden Säureanion

thermisch oberflächennachvernetzt werden, **dadurch gekennzeichnet, dass** die oberflächennachvernetzten Polymerpartikel anschließend nachbehandelt werden und die Nachbehandlung die Schritte

i) Beschichtung mit mindestens einem Salz aus einem polyvalenten Metallkation und einem nicht-komplexierenden Säureanion,
ii) Erhöhung des Feuchtgehalts um 1 bis 150 Gew.-% und
iii) Trocknung nach der Erhöhung des Feuchtgehalts

umfasst.

2.  Verfahren gemäß Anspruch 1, **dadurch gekennzeichnet, dass** der Schritt ii) vor dem Schritt i) durchgeführt wird.

3.  Verfahren gemäß Anspruch 1 oder 2, **dadurch gekennzeichnet, dass** das komplexierende Säureanion ausgewählt ist aus der Gruppe Glykolat, Glycinat, Laktat, Alanat, Citrat, Tartrat, Tartronat und Glycerat.

4.  Verfahren gemäß einem der Ansprüche 1 bis 3, **dadurch gekennzeichnet, dass** die klassierten Polymerpartikel mit 0,02 bis 0,8 Gew.-% des polyvalenten Metallkations beschichtet werden.

5.  Verfahren gemäß einem der Ansprüche 1 bis 4, **dadurch gekennzeichnet, dass** das nicht-komplexierende Säureanion ausgewählt ist aus der Gruppe Formiat, Acetat, Propionat, Methylsulfonat, Sulfat und Chlorid.

6.  Verfahren gemäß einem der Ansprüche 1 bis 5, **dadurch gekennzeichnet, dass** die oberflächennachvernetzten Polymerpartikel mit 0,02 bis 0,8 Gew.-% des polyvalenten Metallkations beschichtet werden.

7.  Verfahren gemäß einem der Ansprüche 1 bis 6, **dadurch gekennzeichnet, dass** das polyvalente Metallkation ausgewählt ist aus der Gruppe Al3+, Ti4+ und Zr4+.

8.  Verfahren gemäß einem der Ansprüche 1 bis 7, **dadurch gekennzeichnet, dass** die oberflächennachvernetzten Polymerpartikel nach Erhöhung des Feuchtegehalts bei einer Temperatur von weniger als 150°C getrocknet werden.

9.  Verfahren gemäß einem der Ansprüche 1 bis 8, **dadurch gekennzeichnet, dass** die oberflächennachvernetzten Polymerpartikel nach Erhöhung des Feuchtegehalts bis zu einem Feuchtegehalt von weniger als 10 Gew.-% getrocknet werden.

10. Verfahren gemäß einem der Ansprüche 1 bis 9, **dadurch gekennzeichnet, dass** die wasserabsorbierenden Polymerpartikel eine Zentrifugenretentionskapazität von mindestens 15 g/g aufweisen.

11. Wasserabsorbierende Polymerpartikel, erhältlich durch Polymerisation einer Monomerlösung oder -suspension zu einem Polymergel, Trocknung, Mahlung und Klassierung des Polymergels zu Polymerpartikeln und thermischer Oberflächennachvernetzung der Polymerpartikel, wobei die wasserabsorbierenden Polymerpartikel einen Feuchtegehalt von weniger als 10 Gew.-%, eine Zentrifugenretentionskapazität von mindestens 15 g/g, eine Flüssigkeitsweiterleitung von mindestens $100{\times}10^{-7}$cm$^3$s/g, eine Gelbettpermeabilität von mindestens 30 Darcies und einen Vortex von weniger als 70s aufweisen.

12. Polymerpartikel gemäß Anspruch 11, wobei die wasserabsorbierenden Polymerpartikel eine Flüssigkeitsweiterleitung von mindestens $150{\times}10^{-7}$ cm$^3$s/g aufweisen.

13. Polymerpartikel gemäß Anspruch 11 oder 12, wobei die wasserabsorbierenden Polymerpartikel eine Gelbettpermeabilität von mindestens 50 Darcies aufweisen.

14. Polymerpartikel gemäß einem der Ansprüche 10 bis 13, wobei die wasserabsorbierenden Polymerpartikel einen Vortex von weniger als 60s aufweisen.

15. Hygieneartikel, enthaltend wasserabsorbierende Polymerpartikel gemäß einem der Ansprüche 11 bis 14.

**Claims**

1. A process for producing water-absorbing polymer particles by polymerizing a monomer solution or suspension comprising

 a) at least one ethylenically unsaturated monomer which bears acid groups and may be at least partly neutralized,
 b) at least one crosslinker,
 c) at least one initiator,
 d) optionally one or more ethylenically unsaturated monomers copolymerizable with the monomers mentioned under a) and
 e) optionally one or more water-soluble polymers,
 by drying, grinding and classifying the resulting polymer gel, and thermally surface postcrosslinking the classified polymer particles with

 f) at least one covalent surface postcrosslinker and
 g) at least one salt of a polyvalent metal cation and a complexing acid anion,

 which comprises subsequently aftertreating the surface postcrosslinked polymer particles, the aftertreatment comprising the steps of

 i) coating with at least one salt of a polyvalent metal cation and a non-complexing acid anion,
 ii) increasing the moisture content by 1 to 150% by weight and
 iii) drying after the increase in the moisture content.

2. The process according to claim 1, wherein step ii) is conducted before step i).

3. The process according to claim 1 or 2, wherein the complexing acid anion is selected from the group of glycolate, glycinate, lactate, alanate, citrate, tartrate, tartronate and glycerate.

4. The process according to any of claims 1 to 3, wherein the classified polymer particles are coated with 0.02 to 0.8% by weight of the polyvalent metal cation.

5. The process according to any of claims 1 to 4, wherein the non-complexing acid anion is selected from the group of formate, acetate, propionate, methylsulfonate, sulfate and chloride.

6. The process according to any of claims 1 to 5, wherein the surface postcrosslinked polymer particles are coated with 0.02 to 0.8% by weight of the polyvalent metal cation.

7. The process according to any of claims 1 to 6, wherein the polyvalent metal cation is selected from the group of Al3+, Ti4+ and Zr4+.

8. The process according to any of claims 1 to 7, wherein the surface postcrosslinked polymer particles, after increasing the moisture content, are dried at a temperature of less than 150°C.

9. The process according to any of claims 1 to 8, wherein the surface postcrosslinked polymer particles, after increasing the moisture content, are dried down to a moisture content of less than 10% by weight.

10. The process according to any of claims 1 to 9, wherein the water-absorbing polymer particles have a centrifuge retention capacity of at least 15 g/g.

11. Water-absorbing polymer particles obtainable by polymerizing a monomer solution or suspension to give a polymer gel, drying, grinding and classifying the polymer gel to give polymer particles and thermally surface postcrosslinking the polymer particles, the water-absorbing polymer particles having a moisture content of less than 10% by weight, a centrifuge retention capacity of at least 15 g/g, a saline flow conductivity of at least $100 \times 10^{-7}$ cm$^3$s/g, a gel bed permeability of at least 30 darcies and a vortex of less than 70 s.

12. Polymer particles according to claim 11, wherein the water-absorbing polymer particles have a saline flow conductivity of at least $150 \times 10^{-7}$ cm$^3$s/g.

**13.** Polymer particles according to claim 11 or 12, wherein the water-absorbing polymer particles have a gel bed permeability of at least 50 darcies.

**14.** Polymer particles according to any of claims 10 to 13, wherein the water-absorbing polymer particles have a vortex of less than 60 s.

**15.** A hygiene article comprising water-absorbing polymer particles according to any of claims 11 to 14.

**Revendications**

**1.** Procédé de fabrication de particules polymères absorbant l'eau par polymérisation d'une solution ou d'une suspension de monomères, contenant

a) au moins un monomère à insaturation éthylénique, portant des groupes acides, qui peut au moins partiellement être neutralisé,
b) au moins un agent de réticulation,
c) au moins un amorceur,
d) au moins un ou plusieurs monomères à insaturation éthylénique copolymérisables avec les monomères mentionnés en a) et
e) éventuellement un ou plusieurs polymères solubles dans l'eau,
le gel polymère obtenu étant séché, broyé et classé, les particules polymères classées étant soumises à une post-réticulation thermique en surface avec

f) au moins un agent covalent de post-réticulation en surface et
g) au moins un sel d'un cation métallique polyvalent et d'un anion acide complexant,

**caractérisé en ce que** les particules polymères post-réticulées en surface sont ensuite soumises à un post-traitement, et le post-traitement comprend les étapes

i) revêtement avec au moins un sel d'un cation métallique polyvalent et d'un anion acide non complexant,
ii) augmentation de la teneur en humidité de 1 à 150 % en poids et
iii) séchage après augmentation de la teneur en humidité.

**2.** Procédé selon la revendication 1, **caractérisé en ce que** l'étape ii) est mise en œuvre avant l'étape i).

**3.** Procédé selon la revendication 1 ou 2, **caractérisé en ce que** l'anion acide complexant est choisi dans le groupe glycolate, glycinate, lactate, alanate, citrate, tartrate, tartronate et glycérate.

**4.** Procédé selon l'une des revendications 1 à 3, **caractérisé en ce que** les particules polymères classées sont revêtues de 0,02 à 0,8 % en poids du cation métallique polyvalent.

**5.** Procédé selon l'une des revendications 1 à 4, **caractérisé en ce que** l'anion acide non complexant est choisi dans le groupe formiate, acétate, propionate, méthylsulfonate, sulfate et chlorure.

**6.** Procédé selon l'une des revendications 1 à 5, **caractérisé en ce que** les particules polymères post-réticulées en surface sont revêtues avec 0,02 à 0,8 % en poids du cation métallique polyvalent.

**7.** Procédé selon l'une des revendications 1 à 6, **caractérisé en ce que** le cation métallique polyvalent est choisi dans le groupe Al3+, Ti4+ et Zr4+.

**8.** Procédé selon l'une des revendications 1 à 7, **caractérisé en ce que** les particules polymères post-réticulées en surface sont, après augmentation de la teneur en humidité, séchées à une température inférieure à 150 °C.

**9.** Procédé selon l'une des revendications 1 à 8, **caractérisé en ce que** les particules polymères post-réticulées en surface sont, après augmentation de la teneur en humidité, séchées jusqu'à une teneur en humidité inférieure à 10 % en poids.

**10.** Procédé selon l'une des revendications 1 à 9, **caractérisé en ce que** les particules polymères absorbant l'eau présentent un pouvoir de rétention après centrifugation d'au moins 15 g/g.

**11.** Particules polymères absorbant l'eau pouvant être obtenues par polymérisation d'une solution ou d'une suspension de monomères conduisant à un gel polymère, séchage, broyage et classement du gel polymère en des particules polymères et post-réticulation thermique en surface des particules polymères, les particules polymères absorbant l'eau présentant une teneur en humidité inférieure à 10 % en poids, un pouvoir de rétention après centrifugation d'au moins 15 g/g, un transfert de liquide d'au moins $80 \times 10^{-7}$ cm$^3$s/g, une perméabilité du lit de gel d'au moins 30 darcys et un vortex inférieur à 70 secondes.

**12.** Particules polymères selon la revendication 11, les particules polymères absorbant l'eau présentant un transfert de liquide d'au moins $150 \times 10^{-7}$ cm$^3$s/g.

**13.** Particules polymères selon la revendication 11 ou 12, les particules polymères absorbant l'eau présentant une perméabilité du lit de gel d'au moins 50 darcys.

**14.** Particules polymères selon l'une des revendications 10 à 13, les particules polymères absorbant l'eau présentant un vortex inférieur à 60 s.

**15.** Article hygiénique contenant des particules polymères absorbant l'eau selon l'une des revendications 11 à 14.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012045705 A1
- WO 2012107432 A1
- WO 2010149735 A1
- US 2011059329 A1
- WO 2010057912 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1
- US 20050256757 A
- WO 0138402 A1
- US 20050176910 A

**In der Beschreibung aufgeführte Nicht-Patentliteratur**

- **F.L. BUCHHOLZ** ; **A.T. GRAHAM**. Modern Superabsorbent Polymer Technology. Wiley-VCH, 1998, 71-103
- Standard Test Methods for the Nonwovens Industry. 2005